Europäisches Patentamt

European Patent Office

Office européen des brevets

(1) Veröffentlichungsnummer: **0 359 124**
**A2**

## EUROPÄISCHE PATENTANMELDUNG

(21) Anmeldenummer: 89116535.9

(22) Anmeldetag: 07.09.89

(51) Int. Cl.5: **H01J 49/04**

(30) Priorität: 14.09.88 DE 3831258

(43) Veröffentlichungstag der Anmeldung:
21.03.90 Patentblatt 90/12

(84) Benannte Vertragsstaaten:
**AT CH DE FR GB IT LI LU NL SE**

(71) Anmelder: **ALCATEL HOCHVAKUUMTECHNIK GmbH**
**Am Kreuzeck 10**
**D-6980 Wertheim/Main(DE)**

(72) Erfinder: **Bürger, Heinz Dieter**
**Breslauer Strasse 111**
**D-6980 Wertheim/Main(DE)**

(74) Vertreter: **Weinmiller, Jürgen**
**Lennéstrasse 9 Postfach 24**
**D-8133 Feldafing(DE)**

(54) Verfahren zur Gasanalyse nach dem Gegenstromprinzip.

(57) Die Erfindung betrifft ein Verfahren zur Gasanalyse nach dem Gegenstromprinzip, mit einer Massenspektrometerröhre (3) , die an der Saugseite einer Molekularpumpe (2) liegt, an deren Auslaßseite eine Vorvakuumpumpe (1) sowie ein Einlaß für das zu analysierende Gas angeordnet sind. Erfindungsgemäß wird das Kompressionsverhältnis der Molekularpumpe (2) so eingestellt, daß prinzipiell mehrdeutige Spektrometeranzeigen nur noch von einem Gas niedrigeren Molekulargewichts herrühren können und nicht mehr von einem Gas höheren Molekulargewichts, das an sich eine ähnliche Spektrometeranzeige hervorrufen würde.

FIG. 1

EP 0 359 124 A2

# VERFAHREN ZUR GASANALYSE NACH DEM GEGENSTROMPRINZIP

Die Erfindung bezieht sich auf ein Verfahren zur Gasanalyse nach dem Gegenstromprinzip, mit einer Massenspektrometeröhre, die an der Saugseite einer Molekularpumpe liegt, an deren Auslaßseite eine Vorvakuumpumpe sowie ein Einlaß für das zu analysierende Gas angeordnet sind.

Gasanalyseverfahren mit Massenspektrometer leiden infolge der verwendeten Ionisationsprozesse mit Elektronen an wesentlichen Mängeln, die ihre Anwendbarkeit für verschiedene Problemstellungen stark einschränken bzw. unmöglich machen. Zu diesen Mängeln zählen insbesondere die Fragmentbildung bei der Ionisation, die den Nachweis der ursprünglich vorhandenen Moleküle erschweren, da aus sehr unterschiedlichen Stoffen nahezu identische Fragmente entstehen können.

Beim Ionisationsvorgang werden Elektronen in der Regel auf eine kinetische Energie von mindestens 70 eV gebracht. Bei diesen Energien liegen die Wirkungsquerschnitte bzw. die Ionisierungseffizienz in brauchbaren Größenordnungen, jedoch muß als entscheidender Nachteil die Fragmentierung von Molekülen in Kauf genommen werden. So ergeben sich beispielsweise durch Ionisation mit Elektronen aus Kohlendioxid und Kohlenmonoxid praktisch identische Fragmente, so daß der getrennte Nachweis beider Gase erschwert wird.

In gewissen Grenzen kommt man zu befriedigenden Ergebnissen, wenn man nicht nur die Hauptpeaks des Massenspektrometers auswertet, sondern das ganze Spektrum einschließlich der von den Fragmenten herrührenden Nebenpeaks, d.h. die sogenannten massenspektrometrischen Fingerprints aufnimmt (siehe z.B. die Zeitschrift Vakuum, 16, 67, (1966) "The interpretation of mass spectra in vacuum measurement"). Jedoch liegen manche dieser Fingerprints so nahe beieinander, daß ihre Unterscheidung unmöglich wird. So findet man z.B. bei n-Butan (Masse 58) den Hauptpeak bei Masse 43 und den nächstgrößeren Peak bei Masse 29. Bei Masse 43 erscheint aber auch der Hauptpeak von Aceton und ebenfalls ein Nebenpeak unter anderem bei Masse 29.

Um diese Schwierigkeiten der Interpretation von Spektren zu vermeiden, wurde bereits eine sogenannte Softionisation vorgeschlagen, um die Fragmentbildung möglichst gering zu halten. Man versteht darunter eine Ionisierung in zwei Schritten, wobei in einem ersten Schritt mit Elektronen ein Primärionenschwarm eines Edelgases erzeugt wird. Die Primärionen werden dann mit dem zu untersuchenden Gas gemischt, so daß durch Umladungsreaktionen die verschiedenen Moleküle des zu untersuchenden Gases in entsprechende Ionen konvertieren. Durch eine geeignete Auswahl der Primärionen, deren Ionisierungspotential nur unwesentlich über dem des zu ionisierenden Gases liegt, können die neutralen Moleküle ohne wesentliche Fragmentierung in Ionen übergeführt werden.

Dieses Verfahren ist jedoch für den Routineeinsatz zu kompliziert.

Aufgabe der Erfindung ist es also, ein Verfahren der im Oberbegriff des Anspruchs 1 genannten Art anzugeben, das ohne Softionisierung auskommt und doch in vielen Fällen eine klare Unterscheidung zwischen einander ähnlichen Fingerprints erlaubt.

Diese Aufgabe wird erfindungsgemäß dadurch gelöst, daß gezielt das Kompressionsverhältnis der Molekularpumpe jeweils so ein gestellt wird, daß prinzipiell mehrdeutige Spektrometeranzeigen nur noch von einem Gas niedrigeren Molekulargewichts herrühren können und nicht mehr von einem Gas höheren Molekulargewichts, das an sich eine ähnliches Spektrum hervorrufen würde.

Man macht sich also die Tatsache zunutze, daß bei der Gasanalyse nach dem Gegenstromprinzip bevorzugt nur leichte Moleküle entgegen der Pumprichtung der Molekularpumpe bis zur Analysezelle gelangen. Bei Turbomolekularpumpen ergibt sich eine relativ scharfe Grenze zwischen relativ hohem und niedrigem Kompressionsverhältnis, während Holweckpumpen bei höherem Druck einen größeren Regelbereich aufweisen.

Die Veränderung des Kompressionsverhältnisses der Molekularpumpe kann in einer ersten Ausführungsform des erfindungsgemäßen Verfahrens dadurch erreicht werden, daß der Vorvakuumtotaldruck verändert wird.

In einer anderen Ausführungsform wird das Kompressionsverhältnis durch Veränderung der Drehzahl der Molekularpumpe verändert. Es kann aber auch sinnvoll sein, bei Verwendung einer Holweckpumpe als Molekularpumpe die Breite des Ringspalts oder die Länge der wirksamen Pumpzone durch Verformung der elastisch ausgebildeten Statorwand bzw. durch relative Axialverschiebung zwischen Rotor und Stator zu verändern.

Anhand zweier Figuren wird nachfolgend die Erfindung näher erläutert.

Fig. 1 zeigt eine Vorrichtung, an der das erfindungsgemäße Verfahren anwendbar ist.

Fig. 2 zeigt die Abhängigkeit des Kompressionsverhältnisses einer Turbomolekularpumpe vom Vorvakuumdruck für verschiedene Gase.

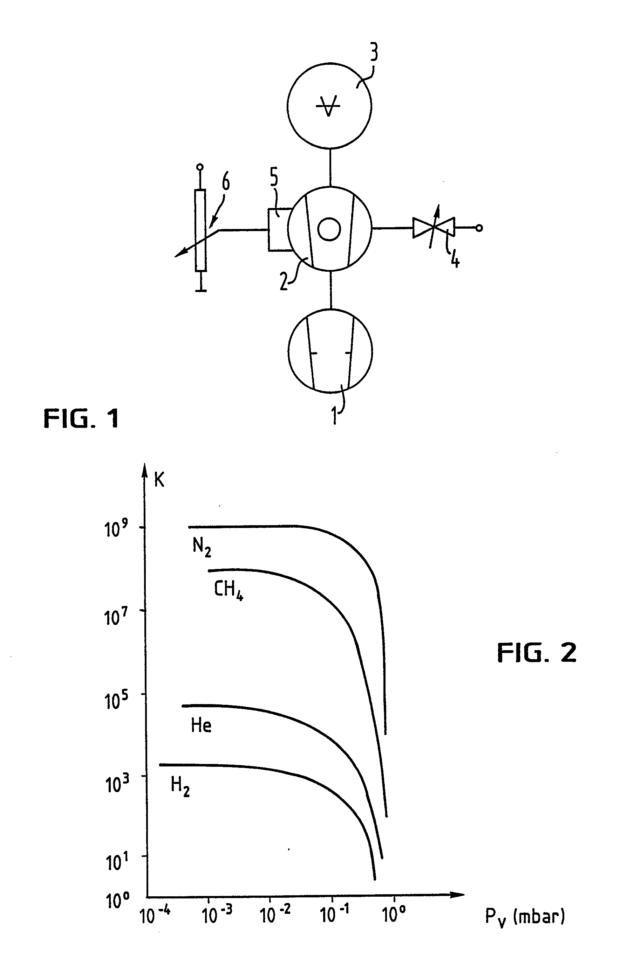

Die Spektrometeranordnung gemäß Fig. 1 besteht aus einem Pumpstand mit einer Vorvakuumpumpe 1 und einer Holweckpumpe 2 sowie aus einer Massenspektrometerzelle 3, die an der Saugseite der Molekularpumpe 2 liegt. An der Drucksei-

2

te dieser Pumpe oder einem Zwischeneinlaß wird über ein Einlaßventil 4 das zu analysierende Gas zugeführt. Die Molekularpumpe 2 wird von einem Motor mit einer in einem Stellglied 6 einstellbaren Drehgeschwindigkeit angetrieben. Es ist bekannt, daß das Kompressionsverhältnis der Molekularpumpe 2 mit der Drehzahl dieser Pumpe variiert. Mit steigender Drehzahl kommen bevorzugt leichte Moleküle entgegen der Pumprichtung bis zur Massenspektrometerzelle. Man kann die Drehzahl also z.B. so festlegen, daß bei einem bestimmten Verhältnis von Partialdruck zu Vorvakuumdruck zwar Propan noch in die Massenspektrometerzelle gelangt, nicht aber Kohlendioxid oder Ameisensäure oder Butan, Aceton, Essigsäure, Pumpenöl usw., die alle ähnliche Fingerprints mit einem Peak bei Masse 43 und einem Peak bei Masse 44 besitzen.

Eine andere Möglichkeit, das Kompressionsverhältnis der Molekularpumpe selektiv zu verändern, besteht in der Veränderung des Vorvakuumdrucks. Fig. 2 zeigt typische Kurven der Abhängigkeit des Kompressionsverhältnisses $K$ vom Vorvakuumdruck $p_v$ (in Millibar) für eine Turbomolekularpumpe. Man erkennt, daß bei hohem Vorvakuumdruck (schlechtem Vorvakuum) Moleküle grösserer Masse durch die Turbomolekularpumpe zur Massenspektrometerzelle diffundieren können als bei einem besseren Vorvakuun.

Weitere Möglichkeiten, die Rückdiffusionsselektivität zu steuern, liegen in der Vergrößerung oder Verkleinerung des Radial spiels zwischen der glatten Wand und der mit Holwecknuten versehenen Wand der Pumpe, derart, daß sich die Spiralnuten im Rotor befinden und die zylindrische Statorwand eine Membran aus elastischem Stahl bildet, deren Durchmesser hydraulisch verändert werden kann. Diese Technik ist z.B. von sogenannten Hydrodehnspannhülsen bekannt.

Andererseits kann das Kompressionsverhältnis der Molekularpumpe auch durch axiale Verschiebung zwischen Stator und Rotor erreicht werden. Hierdurch wird die Pumpzone verkürzt oder verlängert und somit das Kompressionsverhältnis verändert.

## Ansprüche

1. Verfahren zur Gasanalyse nach dem Gegenstromprinzip, mit einer Massenspektrometerröhre, die an der Saugseite einer Molekularpumpe liegt, an deren Auslaßseite eine Vorvakuumpumpe sowie ein Einlaß für das zu analysierende Gas angeordnet sind, dadurch **gekennzeichnet**, daß das Kompressionsverhältnis der Molekularpumpe so eingestellt wird, daß prinzipiell mehrdeutige Spektrometeranzeigen nur noch von einem Gas niedrigeren Molekulargewichts herrühren können und nicht mehr von einem Gas höheren Molekulargewichts, das an sich eine ähnliche Spektrometeranzeige hervorrufen würde.

2. Verfahren nach Anspruch 1, dadurch gekennzeichnet, daß eine Veränderung des Kompressionsverhältnisses der Molekularpumpe dadurch erreicht wird, daß der Vorvakuumtotaldruck verändert wird.

3. Verfahren nach Anspruch 1 oder 2, dadurch gekennzeichnet, daß eine Veränderung des Kompressionsverhältnisses der Molekularpumpe dadurch erreicht wird, daß die Drehzahl der Molekularpumpe verändert wird.

4. Verfahren nach einem der Ansprüche 1 bis 3, dadurch gekennzeichnet, daß eine Veränderung des Kompressionsverhältnisses der als Holweckpumpe ausgebildeten Molekularpumpe dadurch erreicht wird, daß die Breite des Ringspalts verändert wird.

5. Verfahren nach einem der Ansprüche 1 bis 3, dadurch gekennzeichnet, daß eine Veränderung des Kompressionsverhältnisses der als Holweckpumpe ausgebildeten Molekularpumpe dadurch erreicht wird, daß die Länge der Pumpzone durch relative Axialverschiebung zwischen Rotor und Stator verändert wird.

**FIG. 1**

**FIG. 2**